# EUROPEAN PATENT SPECIFICATION

(11) **EP 2 605 760 B1**
(45) Date of publication and mention of the grant of the patent: **11.01.2017**
(21) Application number: 11751736.7
(22) Date of filing: 17.08.2011
(51) Int. Cl.: A61K 9/28, A61K 9/20

(54) **TABLET SLEEVE FOR IMPROVED PERFORMANCE**
TABLETTEHÜLLE FÜR OPTIMIERTE LEISTUNG
GAINE DE COMPRIMÉ POUR PERFORMANCE AMÉLIORÉE

(30) Priority: 18.08.2010 US 374675 P
(43) Date of publication of application: 26.06.2013
(73) Proprietor: Johnson & Johnson Consumer Inc., Skillman, NJ 08558 (US)
(72) Inventor: WALDMAN, Joel, Chalfont, PA 18914 (US)
(74) Representative: Carpmaels & Ransford LLP
(86) International application number: PCT/US2011/048024
(87) International publication number: WO 2012/024360

(56) References cited:
- EP-A2- 1 213 014
- US-A- 5 658 589
- US-A- 5 667 804
- US-A- 6 120 803
- US-A1- 2005 152 970

## Description

### FIELD OF THE INVENTION

The present invention relates to pharmaceutical compositions and more particularly to sleeve coated pharmaceutical compositions having enhanced swallowability and appearance. The present invention also relates to a method and apparatus for preparing the sleeve coated pharmaceutical compositions.

### BACKGROUND

It is known to coat tablets with a polymer or gelatin. Such coatings have been applied with empty capsule shells or other methods such as enrobing, compression coating and dipping. All of these methods involve two step processes since each involves placing one portion of the coating on one end, and another portion of the coating on the opposite end.

For example, U.S. Patents Nos. 5,415,868 and 6,126,767 to L. Perrigo Company disclose a method of covering a caplet with a gelatin cover that includes applying pharmaceutically acceptable adhesive to first and second ends of the caplet, and then placing hard-shell gelatin capsule halves on the first and second ends of the caplet.

U.S. Patent No. 6,080,426 to Warner Lambert Company discloses caplets encapsulated in a capsule, wherein an adhesive is sprayed onto the surface of the caplet and/or onto the inner surface of the capsule immediately before assembling.

U.S. Patent No. 5,464,631 to Warner Lambert Company discloses a tamper resistant and tamper evident dosage form. The dosage form comprises an active pharmaceutical ingredient, pharmaceutically acceptable carrier materials and excipients that are compressed into a generally ovoid, cylindrically shaped caplet that is partially encapsulated by and adhesively bonded to a gelatin capsule. The caplet is of a first color and the gelatin capsule is of a second, different color.

U.S. Patent No. 5,188,688 to Minnesota Mining and Manufacturing Company discloses the use of a water soluble, amide-containing polymer adhesive in a volatile, essentially nonaqueous solvent to seal gelatin capsule sections together at less than the entire circumference of overlap between capsule sections. The sealant may also adhere a pharmaceutical caplet within the capsule to the internal wall of the capsule. The method of applying the sealant to the capsule sections and possibly also the pharmaceutical caplet uses a drop of the sealant spread at the junction of an eccentric arcuate portion of the sections and the pharmaceutical caplet. The solvent evaporates from the sealant through the portion of the capsule section overlap not sealed.

U.S. Patent No. 4,928,840 to American Home Products Corp. discloses a tamper proof encapsulated dosage form that comprises a caplet adhesively bonded to the inner end surfaces of an elongated essentially round gelatin capsule such that upon tampering the dosage form will be destroyed or rendered non-reusable. The gelatin capsule is made in two slip fit joinable sections.

U.S. Patent No. 6,482,516 to Banner Pharmacaps, Inc. discloses a gelatin film enrobed caplet that comprises a core and a gelatin coating that fully encloses the core. The coating is defined by layers of soft elastic gelatin film which are applied to opposite sides of the core and sealed together.

U.S. Patent No. 6,018,935 to Perrone discloses a machine for enrobing tablets with gelatin.

U.S. Patent No. 5,460,824 to Warner-Lambert Company discloses a method for producing medicine that comprises inserting the medicine in a first and a second portion of a two-part capsule and applying energy in the form of heat to shrink the capsule around the medicine.

U.S. Patent No. 5,511,361 to Warner-Lambert Company discloses a method for encapsulating medicine that comprises fitting gelatin half capsules over opposite ends of the medicine.

U.S. Patent No. 4,928,813 to Olin Corporation discloses a capsule for use in the dissolution of a halogen chemical compound into a body of water that comprises a tablet section, shell means fitted about the tablet section, wherein the shell means has an elongate center portion substantially encasing the tablet section and two end portions adjacent tablet end sections. The two end portions generally extend away from the tablet section in substantially opposite directions from the ends of the tablet. The extension sections each comprise a conduit wall forming a conduit between apertures and the exterior of the capsule.

US 2005/0152970 A relates to a gelatinous coated dosage form having two regions coated with gelatinous materials and an exposed circumferential band. Openings are provided in at least the exposed band to reveal the core material.

There is a need in the art for improved dosage forms for improvement of swallowability associated with gelatin coated tablets, and improvement of dissolution in tablets with gelatin coatings.

### SUMMARY OF THE INVENTION

It is an object of this invention to provide a sleeve coated dosage form capable of being provided in one, two or more colors.

It is another object of this invention to provide a sleeve coated dosage form that is tamper-resistant.

It is another object of this invention to provide a sleeve coated dosage form that provides greater ease in swallowing and is perceived to be more effective than pan-coated or spray coated pharmaceutical equivalents, capsules, or coated particulates.

It is still another object of this invention to provide a novel and less expensive method and apparatus for adapting existing equipment for manufacturing coated caplets.

It is still another object of this invention to provide coating to cover imperfections inherent on the caplet core.

It is an object of the present invention to provide a tamper-resistant pharmaceutical capsule whereby a medicament in the form of a caplet, is coated with a sleeve coating which is essentially tasteless and easy to swallow.

With these and other objects in view, which will become apparent to one skilled in the art as the description proceeds, this invention resides in the novel construction, combination, arrangements of parts and methods substantially as hereinafter described and more particularly defined by the attached claims.

### DETAILED DESCRIPTION OF THE INVENTION

The present invention relates to pharmaceutical compositions and more particularly to sleeve coated pharmaceutical compositions as defined in claim 1 having enhanced swallowability and appearance.

The present invention provides a novel method for coating solid cores, such as caplets, with coatings to produce capsule-like dosage forms. Such capsule-like dosage forms are achieved by applying a sleeve over the caplet to provide a dosage form which is observed to be similar to a regular capsule, or a capsule with partial coating on the ends of such capsule. The production of such caplets is readily facilitated by simple and inexpensive modifications to existing production equipment or by similarly designed newer equipment. In particular, the preferred apparatus of the present invention replaces the prior art standard production dipping machine or capsule filling maching with novel caplet holding means having caplet channels therein for receiving, individually gripping and adding a sleeve to said caplet. Also included, are novel sleeve coating designs which readily facilitate the method of this invention. As a result, novel capsule-like dosage forms are provided having smooth, relatively thick, shiny, multicolored coatings thereon. These dosage forms are pleasing to the eye, and should be perceived by consumers as easier to swallow and more effective than prior art caplet dosage forms, while providing much greater tamper resistance than conventional capsules.

As used herein, the term "dosage form" applies to any solid object, semi-solid, or liquid-filled composition designed to contain a specific pre-determined amount (dose) of a certain ingredient, for example, an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components provided that the liquid or semi-liquid is enveloped or surrounded by another material to enable it to hold a form. The liquid or semi-solid filled form may be a soft gelatin capsule or a hard shell gelatin capsule which further comprises the tablet sleeve. In a particularly preferred embodiment, the dosage form is an orally administered system for delivering a pharmaceutical active ingredient to the gastro-intestinal tract of a human.

Suitable "active ingredients" for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof. Suitable oral care agents include breath fresheners, tooth whiteners, antimicrobial agents, tooth mineralizers, tooth decay inhibitors, topical anesthetics, mucoprotectants, and the like. Suitable flavorants include sweeteners, menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine. A preferred gastrointestinal agent is omeprazole.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics, e.g., non-steroidal anti-inflammatory drugs (NSAIDs), including propionic acid derivatives, e.g., ibuprofen, naproxen, ketoprofen and the like; acetic acid derivatives, e.g., indomethacin, diclofenac, sulindac, tolmetin, and the like; fenamic acid derivatives, e.g., mefanamic acid, meclofenamic acid, flufenamic acid, and the like; biphenylcarbodylic acid derivatives, e.g., diflunisal, flufenisal, and the like; and oxicams, e.g., piroxicam, sudoxicam, isoxicam, meloxicam, and the like. In one particular embodiment, the active ingredient is selected from propionic acid derivative NSAID, e.g., ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof. A preferred (analgesic) is acetaminophen.

In another embodiment of the invention, the active ingredient may be selected from upper respiratory agents, such as pseudoephedrine, phenylephrine, guaifensin, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof. A preferred upper respiratory agent is phenylephrine HCl. Another preferred upper respiratory agent is guaifensin.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises at least about 1 weight percent, for example, the dosage form comprises at least about 5 weight percent, say at least about 20 weight percent, of a combination of one or more active ingredients. In one embodiment, a core comprises a total of at least about 25 weight percent (based on the weight of the core) of one or more active ingredients.

The active ingredient or ingredients may be present in the dosage form in any form. For example, the active ingredient may be dispersed at the molecular level, e.g., melted or dissolved, within the dosage form, or may be in the form of particles, which in turn may be coated or uncoated. If an active ingredient is in the form of particles, the particles (whether coated or uncoated) typically have an average particle size of about 1-2000 microns. In one preferred embodiment, such particles are crystals having an average particle size of about 1-300 microns. In another preferred embodiment, the particles are granules or pellets having an average particle size of about 50-2000 microns, preferably about 50-1000 microns, most preferably about 100-800 microns.

Each core may be any solid form. As used herein, "core" refers to a material which is at least partially enveloped or surrounded by another material and has a thickness of at least about 2 mm to about 30 mm. Preferably, a core is a self-contained unitary object, such as a "tablet", which is a compressed or molded solid dosage form of any size or shape. Solid, generally oblong-shaped tablets may sometimes be referred to as "caplets".

The cores may be prepared by any suitable method, including for example compression or molding, and depending on the method by which they are made, typically comprise active ingredient and a variety of excipients. In embodiments in which the core is made by compression, suitable excipients include fillers, binders, disintegrants, lubricants, glidants, and the like, as known in the art.

Suitable fillers for use in making a core or core portion by compression include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, tricalcium phosphate and the like and mixtures thereof.

Suitable binders for making a core or core portion by compression include dry binders such as polyvinyl pyrrolidone, hydroxypropylcellulose, hydroxypropylmethylcellulose, and the like; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, polyvinyl pyrrolidone, cellulosics, sucrose, starches, and the like; and derivatives and mixtures thereof.

Suitable disintegrants for making a core or core portion by compression include sodium starch glycolate, cross-linked polyvinylpyrrolidone, cross-linked carboxymethylcellulose, starches, microcrystalline cellulose, crospovidone and the like.

Suitable lubricants for making a core or core portion by compression include long chain fatty acids and their salts, such as magnesium stearate and stearic acid, talc, glycerides and waxes.

Suitable colorants include lakes, dyes, and opacifiers, including metal containing lakes such as aluminum, magnesium and calcium lakes. Specific opacifiers include but are not limited to titanium dioxide.

Suitable glidants for making a core or core portion by compression, include colloidal silicon dioxide, and the like.

Suitable pH-dependent polymers for use as release-modifying excipients for making a core or core portion by compression include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S, and poly(methacrylic acid, methyl methacrylate) 1:1, which is commercially available from Rohm Pharma GmbH under the tradename EUDRAGIT L, and the like, and derivatives, salts, copolymers, and combinations thereof. In one embodiment a pH dependent polymer is applied to the core containing the active ingredient in a first coating step. In one embodiment this first coating step substantially surrounds the core. As defined herein, "substantially surrounds" includes covering at least 95%, e.g., at least 99% of the surface area of the core. This first coating step may be applied by any method; including spraying, compression coating, enrobing or dipping.

The present invention provides a novel method for coating caplets with coatings to produce capsule-like dosage forms. The subject method may be performed by modifying existing machines or by newer similarly designed apparatus.

The novel process of this invention comprises the steps of providing a holding means having a caplet channel defined therein and inserting a first end of a caplet into said caplet channel while leaving the second end of the caplet exposed. The holding means is then manipulated relative to an open ended sleeve to place the open-ended sleeve on the caplet. The resulting open-ended sleeve on the caplet is then permitted, and preferably caused, to cure to form a coated caplet.

Existing manufacturing may be readily adapted for the purpose of producing the coated caplet products of the present invention. For example, as disclosed in U.S. Patent No. 5,314,537 to Berta, conventional bars of such machines having stainless steel capsule-forming protuberances mounted thereon can be replaced with bars having a plurality of cylindrical holding means mounted thereon. Each holding means receives, retains and facilitates the transfer of an individual caplet. The apparatus can be fitted with a caplet feeder to feed caplets into each holding means. The holding means may, for example, be a cylinder which is open at both ends and which comprises a retaining means, such as "O"-rings or a spring biased retainer for the purpose of holding each caplet in position during the open ended sleeve process. The feeding means may be associated with an inserting means, which may be a simple channel and plunger assembly, for inserting a first end of each caplet into an appropriate holding means. The feeding means ensures that each caplet is inserted a sufficient distance to cause the second end of the caplet to appropriately protrude there from during the upcoming open-ended sleeve coating process. Once each bar is loaded with caplets, it then proceeds to a station where the open-ended sleeve coatings are applied to the exposed ends of the caplets protruding there from.

The "sleeve" may be composed of natural or artificial material that is capable of producing a thin film, including but not limited to, gelatin, carageenan, HPMC, starch, polymethacrylates, or combinations thereof.

The sleeve may be friction fit onto the caplet, held in place with suitable adhesive material and/or shrink fit through the application of heat or other curing process. The sleeve may be pre-formed as a sleeve for a single tablet, trimmed from an extended tube at the time of application, and/or formed from a film or ribbon of material at the time of application. In one embodiment, the sleeve is added using ink, wherein a roller applies the ink to the center portion of the caplet, allowing a differentiated bilateral color, with different colors on the ends.

The sleeve may be unprinted, pre-printed or printed in-situ. The sleeve may be solid or perforated with decorative or functional designs.

In one embodiment the sleeve is raised at least about 1 mm above the surface of the core. The tablet sleeve of the invention provides advantages, including, for example:
- The presence of the sleeve on the tablet surface may improve the swallowability of a coated or uncoated caplet core.
- Taste masking of unpleasant or undesirable flavors from the tablet surface may be accomplished due to the reduced surface area available for interaction with the oral cavity.
- Decreased coating thickness may be achieved due to secondary protection from the sleeve.
- Reduced coating thickness may facilitate the release of active pharmaceutical ingredients from the tablet core.
- The sleeve may take the place of tablet coating for tablets unable to be coated due to physical strength or chemical incompatibility.
- Drug release profile from the dosage form may be modified through use of sleeves with specific permeability or solubility properties.
- Improved dissolution over gelatin coated tablets or caplets that are fully coated, and wherein the gelatin may crosslink upon aging or in accelerated conditions.

Prior art processes require bilateral application of end caps to the tablet. The process of the invention allows unilateral application to the tablet surface.

Single or multiple sleeves may be applied to the same tablet from either or both ends for unique visual or functional effects.

In one embodiment an adhesive may be added to the inside walls of the sleeve or to the surface of the core. In one embodiment this adhesive is a polymer suitable for oral pharmaceutical dosage forms, such as but not limited to povidone, hypromellose, hydroxypropyl cellulose, methylcellulose, or starch. In another embodiment the adhesive is a sugar or sugar alcohol including but not limited to sucrose, dextrose, mannitol, sorbitol, xylitol, xylose, and mannose. In one embodiment the adhesive is a material which melts below 100 degrees celcius, e.g., a meltable adhesive, such as polyetheylene glycol, a wax, glyceryl monostearate or glyceryl behenate.

In one embodiment the adhesive is applied by dissolving in a suitable aqueous or organic solvent and spraying onto the surface or the core or the inside surface of sleeve prior to the addition of the sleeve. In order to remove the solvent, the dosage form may be heated in order to remove the solvent. In the case wherein a meltable adhesive is used, the dosage form can be heated after the application of the adhesive and cooled in order to solidify the adhesive. In one embodiment, the meltable adhesive is added as a powder to the core surface or the inside surface of the sleeve wall prior to heating. In one embodiment, the core surface or inside surface of the sleeve is sprayed with the melted form of the meltable adhesive, just prior to the addition of the sleeve to the core.

In one embodiment, the dosage form may comprise a subcoat. Any composition suitable for film-coating a tablet may be used as a subcoating according to the present invention. Examples of suitable subcoatings are disclosed in, e.g., United States Patents Nos. 4,683,256, 4,543,370, 4,643,894, 4,828,841, 4,725,441, 4,802,924, 5,630,871, and 6,274,162. Suitable compositions for use as subcoatings include those manufactured by Colorcon, a division of Berwind Pharmaceutical Services, Inc., 415 Moyer Blvd., West Point, PA 19486 under the tradename "OPADRY^{®}" (a dry concentrate comprising film forming polymer and optionally plasticizer, colorant, and other useful excipients). Additional suitable subcoatings include one or more of the following ingredients: cellulose ethers such as hydroxypropylmethylcellulose, hydroxypropylcellulose, and hydroxyethylcellulose; polycarbohydrates such as xanthan gum, starch, and maltodextrin; plasticizers including for example, glycerin, polyethylene glycol, propylene glycol, dibutyl sebecate, triethyl citrate, vegetable oils such as castor oil, surfactants such as Polysorbate-80, sodium lauryl sulfate and dioctyl-sodium sulfosuccinate; polycarbohydrates, pigments, and opacifiers.

In one embodiment, the subcoating comprises from about 2 percent to about 8 percent, e.g., from about 4 percent to about 6 percent, of a water-soluble cellulose ether and from about 0.1 percent to about 1 percent, castor oil, as disclosed in United States Patent No. 5,658, 589, In another embodiment, the subcoating comprises from about 20 percent to about 50 percent, e.g., from about 25 percent to about 40 percent, of HPMC; from about 45 percent to about 75 percent, e.g., from about 50 percent to about 70 percent, of maltodextrin; and from about 1 percent to about 10 percent, e.g., from about 5 percent to about 10 percent, of PEG 400. The dried subcoating typically is present in an amount, based upon the dry weight of the core, from about 0 percent to about 5 percent. The subcoating is typically provided by spraying in a coating pan or fluidized bed to cover the tablet in a conventional manner. The subcoating is optionally tinted or colored with colorants such as pigments, dyes and mixtures thereof.

### Tablet Sleeve

In one embodiment, the tablet sleeve is preferably comprised of gelatin. Gelatins have traditionally served as a primary dip-coating material. Hence, the phrase "gelatinous" material. Recently, further work has been done to expand the range of materials capable of providing the desired glossy finish that contain substantially no gelatins.

Gelatin is a natural, thermogelling polymer. It is a tasteless and colorless mixture of derived proteins of the albuminous class, which is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10°C for 17 hours.

In one embodiment, the tablet sleeve is comprised of a material or a combination of materials which do not comprise gelatin. In this embodiment the tablet sleeve is a polymer, a starch, another hydrocolloid, or combinations thereof. Suitable polymers may comprise but are not limited to hypromellose, hydroxypropylcellulose methylcellulose, pullulan, polyvinylalcohol, polymethacrylate, and mixtures, co-polymers and combinations thereof. Suitable starches include but are not limited to modified starches and cornstarch. Suitable hydrocolloids include but are not limited to gellan gum, xanthan gum, guar gum, carageenan, and mixtures thereof.

In one embodiment, the tablet sleeve is free or substantially free of gelatin. As used herein, "substantially gelatin-free" shall mean less than about 1 percent, e.g., less than about 0.5 percent, of gelatin in the composition, and "substantially free of thickeners" shall mean less than about 1 percent, e.g., less than about 0.01 percent, of thickeners in the composition.

In one embodiment, the tablet sleeve comprises a plasticizer. The tablet sleeve may comprise from about 3 percent to about 70 percent of a plasticizer selected from the group consisting of triacetin, acetylated monoglyceride, rape oil, olive oil, sesame oil, acetyltributyl citrate, glycerin sorbitol, diethyloxalate, diethylmalate, diethyl fumarate, dibutyl succinate, diethylmalonate, dioctylphthalate, dibutylsuccinate, triethylcitrate, tributylcitrate, glyceroltributyrate, propylene glycol, polyethylene glycols, hydrogenated castor oil, fatty acids, substituted triglycerides and glycerides, methyl paraben, ethyl paraben, propyl paraben, butyl paraben, polyvinylpyrrolidone, polyethylene glycol 300, polyethylene glycol 400, and pharmaceutically acceptable salts thereof and mixtures thereof

In one embodiment, the sleeve may comprise at least one active ingredient. The active ingredient may be present in the sleeve as a suspended particle, or solubilized as part of the solidified sleeve, as a solid solution. In one embodiment the active ingredient in the sleeve is a different active ingredient than the active ingredient contained in the core tablet. In one embodiment the active ingredient in the sleeve is the same as the active ingredient in the core. In one embodiment, the active ingredient in the sleeve is incompatible with the active ingredient in the core. In one embodiment, the active ingredient may include a therapeutic active agent such as a pharmaceutical active agent. In another embodiment, the active ingredient may include a nutritional active agent such as a vitamin or mineral.

In one embodiment, the tablet sleeve comprises at least one flavorant, acidulant, salivation inducing agent, sensate or mixtures thereof. Suitable sensates include, but are not limited to, cooling, warming, and tingling sensates. Suitable acidulants include but are not limited to, citric acid, fumaric acid, malic acid, and ascorbic acid.

The active ingredient, flavorant, acidulant, salivation inducing agent, or sensate may be present in the tablet sleeve in various forms. For example, the pharmaceutically active agent may be dispersed at the molecular level, e.g. melted or solubilized, within the sleeve, or may be in the form of particles, which in turn may be coated or uncoated. If the pharmaceutically active agent is in form of particles, the particles (whether coated or uncoated) typically have an average particle size of from about 1 to about 2000 microns. In one embodiment, such particles are crystals having an average particle size of from about 1 to about 300 microns.

In one embodiment, wherein the active ingredient is contained in the tablet sleeve, the tablet sleeve displays modified or sustained release properties. In one embodiment, the modified release sleeve comprises a pH dependent polymer such as an enteric polymer or reversed enteric polymer. Suitable enteric polymers include but are not limited to cellulose acetate phthalate, polyvinylaectate phthalate, hypromellose phthalate, and anionic polymers of methacrylic acid and methacrylates which dissolve at a pH 5.5 and above ("Eudragit L 100-55";) a 30% dispersion of methylmethacrylate-methacrylic acid copolymers having a carboxylic acid group ("Eudragit L 30;") methylmethacrylate-methacrylic acid copolymers having a carboxylic acid group ("Eudragit L100;") and the anionic copolymer of methacrylic acid with carboxylic acid groups ("Eudragit S100"). In one embodiment the active ingredient in the tablet sleeve is released in a modified release manner and the active ingredient in the core tablet is released in an immediate release manner. In one embodiment, the tablet sleeve is free of a visible seam across the longitudinal or latitudinal axis, which can be indicative of two portions of gelatin, gelatin solution or film being joined together or overlapped.

In one embodiment, the dissolution characteristics of at least one active ingredient follow an "immediate release profile". As used herein, an immediate release profile is one in which the active ingredient dissolves without substantial delay or retardation due to the dosage form. This can be contrasted with the dissolution of modified release, e.g., delayed or controlled release dosage forms known in the art. In one embodiment, the dissolution rate of immediately released active ingredient from the dosage form of the invention is within about 20% of the dissolution rate of the active ingredient from a pure crystalline powder of said active ingredient, e.g., the time for 50%, 75%, 80%, or 90% dissolution of active ingredient from the dosage form is not more than 20% longer than the corresponding time for 50%, 75%, 80%, or 90% dissolution of active ingredient from a pure crystalline powder of said active ingredient. In another embodiment, the dissolution of immediately released active ingredient from the dosage form of the invention meets USP specifications for immediate release tablets, gelcaps, or capsules containing the active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing; and for acetaminophen and codeine phosphate capsules USP 24 specifies that at least 75% of the acetaminophen contained in the dosage form is dissolved within 30 minutes in 900 mL of 0.1 N Hydrochloric acid using USP Apparatus 2 (paddles) at 50 rpm; and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes. See USP 24, 2000 Version, 19 - 20 and 856 (1999). In yet another embodiment, when the immediately released active ingredient is acetaminophen, when tested in 37°C water using USP Apparatus II (paddles) at 50 rpm, at least 80%, preferably at least 85%, of the acetaminophen contained in the dosage form is released therefrom within 30 minutes.

In one embodiment, the tablet sleeve may comprise an additional active ingredient. In one embodiment, the additional active ingredient in the tablet sleeve is less than 50 mg, such as less than 25 mg, such as less than 15 mg.

In one embodiment, the tablet sleeve comprises a transition line or a seam. In one embodiment, the transition line or seam may denote a separation between two or more distinct colors. The transition line or seam may bifurcate the long or short axis of the tablet sleeve.

When applied from a film or ribbon, the sleeve may be cut so that it either fully enrobes the tablet or does not completely enrobe the tablet along specified axes.

Controlled release of pharmaceutical compound is possible through use of insoluble sleeves or sleeves with pH dependent solubility or permeability.

The method of the invention provides reduced cost and complexity from prior techniques.

In view of the above it is expected that a novel, capsule-like dosage form can be produced. The coated dosage form of this invention which can be produced by the above method comprises a solid tablet or caplet a containing a sleeve thereon. The caplet generally is at least 2.5 times longer than it is wide, and ideally comprises a cylindrical shape.

## Claims

1. A dosage form comprising:
a) a core having a center portion, a first end and a second end; and
b) a coating over the center portion of the core;
wherein said coating is comprised of:
1) gelatin; or
2) a polymer, a starch, a hydrocolloid, or combinations thereof;
wherein said polymer comprises hypromellose, hydroxypropylcellulose methylcellulose, pullulan, polyvinylalcohol, polymethacrylate, or mixtures, co-polymers and combinations thereof;
wherein said starch comprises modified starches or cornstarch;
wherein said hydrocolloid comprises gellan gum, xanthan gum, guar gum, carageenan, or mixtures thereof;
wherein a portion of the first end of the core and a portion of the second end of the core are exposed; and
wherein said core comprises at least one active ingredient, and wherein at least 40%, preferably at least 60%, of said at least one active ingredient dissolves within 3 minutes in 900 mLs of water when tested using USP dissolution apparatus II with a paddle speed of 50 rpm.

2. The dosage form of claim 1, further comprising:
a subcoating.

3. The dosage form of claim 2, wherein said subcoating comprises a first color.

4. The dosage form of claim 1, wherein the coating covers at least 50 percent of a surface area of the dosage form.

5. The dosage form of claim 1, wherein the coating extends at least 1 mm above the core.

6. The dosage form of claim 1, wherein the coating comprises at least one opening.

7. The form of claim 1, wherein the core is a compressed tablet.

8. The dosage form of claim 7, wherein the compressed tablet has an elongated shape.

9. The dosage form of claim 2, wherein the subcoating and the coating contain colorants or coloring agents other than white.

10. The dosage form of claim 1, wherein printed matter is provided on an exterior surface of the coating.

11. The dosage form of claim 1, wherein said dosage form provides immediate release of said active ingredient.

12. The dosage form of claim 1, wherein said at least one active ingredient is selected from the group consisting of acetaminophen, acetyl salicylic acid, ibuprofen, naproxen, ketoprofen, flurbiprofen, diclofenac, cyclobenzaprine, pseudoephedrine, phenylpropanolamine, chlorpheniramine, dextromethorphan, diphenhydramine, astemizole, terfenadine, fexofenadine, loratadine, desloratadine, cetirizine, and pharmaceutically acceptable salts, esters, and isomers thereof.

13. The dosage form of claim 1, further comprising:
c) a second coating over at least a second part of the core.

14. The dosage form of claim 13, further comprising:
a subcoating over portions of the exterior surface of the core.

15. The dosage form of claim 14, wherein the subcoating weight gain is less than or equal to 3%, optionally less than or equal to 2.75%.

16. The dosage form according to claim 2 or claim 14, wherein the subcoating comprises a polymeric material and does not contain a gelatin.

## Patentansprüche

1. Dosierungsform, umfassend:
a) einen Kern mit einem zentralen Teil, einem ersten Ende und einem zweiten Ende und
b) einen Überzug über den zentralen Teil des Kerns,
wobei der Überzug Folgendes umfasst:
1) Gelatine oder
2) ein Polymer, eine Stärke, ein Hydrokolloid oder Kombinationen davon,
wobei das Polymer Hypromellose, Hydroxypropylcellulose, Methylcellulose, Pullulan, Polyvinylalkohol, Polymethacrylat oder Mischungen, Co-Polymere oder Kombinationen davon umfasst,
wobei die Stärke modifizierte Stärken oder Maisstärke umfasst,
wobei das Hydrokolloid Gellan, Xanthan, Guaran, Carrageen oder Mischungen davon umfasst,
wobei ein Teil des ersten Endes des Kerns und ein Teil des zweiten Endes des Kerns freiliegen und
wobei der Kern mindestens einen Wirkstoff umfasst und wobei beim Testen mit einem USP-Auflösungsapparat II mit einer Rührgeschwindigkeit von 50 U/min mindestens 40%, vorzugsweise mindestens 60%, des mindestens einen Wirkstoffs innerhalb von 3 Minuten in 900 ml Wasser in Lösung gehen.

2. Dosierungsform nach Anspruch 1, weiterhin umfassend:
einen Unterüberzug.

3. Dosierungsform nach Anspruch 2, wobei der Unterüberzug eine erste Farbe umfasst.

4. Dosierungsform nach Anspruch 1, wobei der Überzug mindestens 50% einer Oberfläche der Dosierungsform abdeckt.

5. Dosierungsform nach Anspruch 1, wobei sich der Überzug mindestens 1 mm über den Kern erstreckt.

6. Dosierungsform nach Anspruch 1, wobei der Überzug mindestens eine Öffnung umfasst.

7. Form nach Anspruch 1, wobei es sich bei dem Kern um eine komprimierte Tablette handelt.

8. Dosierungsform nach Anspruch 7, wobei die komprimierte Tablette eine längliche Form hat.

9. Dosierungsform nach Anspruch 2, wobei der Unterüberzug und der Überzug nicht-weiße Farbstoffe oder Farbmittel enthalten.

10. Dosierungsform nach Anspruch 1, wobei auf der äußeren Oberfläche des Überzugs Gedrucktes bereitgestellt wird.

11. Dosierungsform nach Anspruch 1, wobei die Dosierungsform für eine sofortige Freisetzung des Wirkstoffs sorgt.

12. Dosierungsform nach Anspruch 1, wobei der mindestens eine Wirkstoff aus der aus Acetaminophen, Acetylsalicylsäure, Ibuprofen, Naproxen, Ketoprofen, Flurbiprofen, Diclofenac, Cyclobenzaprin, Pseudoephedrin, Phenylpropanolamin, Chlorpheniramin, Dextromethorphan, Diphenydramin, Astemizol, Terfenadin, Fexofenadin, Loratadin, Desloratadin, Cetirizin und pharmazeutisch unbedenklichen Salzen, Estern und Isomeren davon bestehenden Gruppe ausgewählt ist.

13. Dosierungsform nach Anspruch 1, weiterhin umfassend:
c) einen zweiten Überzug über mindestens einen zweiten Teil des Kerns.

14. Dosierungsform nach Anspruch 13, weiterhin umfassend:
einen Unterüberzug über Teile der äußeren Oberfläche des Kerns.

15. Dosierungsform nach Anspruch 14, wobei die Unterüberzugsgewichtszunahme weniger als oder gleich 3%, gegebenenfalls weniger als oder gleich 2,75%, ist.

16. Dosierungsform nach Anspruch 2 oder Anspruch 14, wobei der Unterüberzug ein polymeres Material umfasst und keine Gelatine enthält.

## Revendications

1. Forme galénique comprenant :
a) un noyau ayant une partie centrale, une première extrémité et une seconde extrémité ; et
b) un revêtement par-dessus la partie centrale du noyau ;
ledit revêtement étant constitué de :
1) de la gélatine ; ou
2) un polymère, un amidon, un hydrocolloïde ou des associations de ceux-ci ;
ledit polymère comprenant de l'hypromellose, de l'hydroxypropylcellulose, de la méthylcellulose, du pullulane, du poly(alcool vinylique), du polyméthacrylate ou des mélanges, copolymères et associations de ceux-ci ;
ledit amidon comprenant des amidons modifiés ou de l'amidon de maïs ;
ledit hydrocolloïde comprenant de la gomme gellane, de la gomme xanthane, de la gomme de guar, du carraghénane ou des mélanges de ceux-ci ;
dans laquelle une partie de la première extrémité du noyau et une partie de la seconde extrémité du noyau sont exposées ; et
dans laquelle ledit noyau comprend au moins un principe actif et au moins 40 %, de préférence au moins 60 %, dudit au moins un principe actif se dissolvant en moins de 3 minutes dans 900 ml d'eau lorsqu'il est testé à l'aide de l'appareil de dissolution de type II selon la pharmacopée américaine avec une vitesse des palettes de 50 tr/min.

2. Forme galénique selon la revendication 1, comprenant en outre :
un sous-revêtement.

3. Forme galénique selon la revendication 2, dans laquelle ledit sous-revêtement comprend une première couleur.

4. Forme galénique selon la revendication 1, le revêtement recouvrant au moins 50 pour cent d'une surface de la forme galénique.

5. Forme galénique selon la revendication 1, dans laquelle le revêtement s'étend sur au moins 1 mm au-dessus du noyau.

6. Forme galénique selon la revendication 1, dans laquelle le revêtement comprend au moins une ouverture.

7. Forme selon la revendication 1, dans laquelle le noyau est un comprimé.

8. Forme galénique selon la revendication 7, dans laquelle le comprimé a une forme allongée.

9. Forme galénique selon la revendication 2, dans laquelle le sous-revêtement et le revêtement contiennent des colorants ou agents colorants autres que du blanc.

10. Forme galénique selon la revendication 1, dans laquelle de la matière imprimée est disposée sur une surface extérieure du revêtement.

11. Forme galénique selon la revendication 1, ladite forme galénique assurant une libération immédiate dudit principe actif.

12. Forme galénique selon la revendication 1, dans laquelle ledit au moins un principe actif est choisi dans le groupe constitué par l'acétaminophène, l'acide acétylsalicylique, l'ibuprofène, le naproxène, le kétoprofène, le flurbiprofène, le diclofénac, la cyclobenzaprine, la pseudoéphédrine, la phénylpropanolamine, la chlorphéniramine, le dextrométhorphane, la diphénhydramine, l'astémizole, la terfénadine, la fexofénadine, la loratadine, la desloratadine, la cétirizine et les sels pharmaceutiquement acceptables, esters et isomères de ceux-ci.

13. Forme galénique selon la revendication 1, comprenant en outre :
c) un second revêtement par-dessus au moins une seconde partie du noyau.

14. Forme galénique selon la revendication 13, comprenant en outre :
un sous-revêtement par-dessus des parties de la surface extérieure du noyau.

15. Forme galénique selon la revendication 14, dans laquelle l'augmentation de poids du sous-revêtement est inférieure ou égale à 3 %, éventuellement inférieure ou égale à 2,75 %.

16. Forme galénique selon la revendication 2 ou la revendication 14, dans laquelle le sous-revêtement comprend un matériau polymère et ne contient pas une gélatine.
